Europäisches Patentamt

European Patent Office

Office européen des brevets

(19)

(11) Numéro de publication: **0 208 578**
**B1**

(12) **FASCICULE DE BREVET EUROPEEN**

(45) Date de publication du fascicule du brevet: **14.03.90**

(21) Numéro de dépôt: **86401258.8**

(22) Date de dépôt: **10.06.86**

(51) Int. Cl.⁵: **A 61 F 2/32,** A 61 F 2/34,
A 61 F 2/36

(54) Prothèse totale d'une articulation telle que la hanche.

(30) Priorité: **10.06.85 FR 8508741**

(43) Date de publication de la demande:
**14.01.87 Bulletin 87/03**

(45) Mention de la délivrance du brevet:
**14.03.90 Bulletin 90/11**

(84) Etats contractants désignés:
**BE DE FR GB IT**

(56) Documents cités:
**FR-A-1 047 640**
**FR-A-2 266 491**
**FR-A-2 289 162**
**FR-A-2 310 120**
**FR-A-2 437 200**
**GB-A-2 126 096**
**US-A-2 947 308**

(73) Titulaire: **Moulin, Jacques François René**
**43 avenue Edouard Vaillant**
**F-18100 Vierzon (FR)**

(72) Inventeur: **Moulin, Jacques François René**
**43 avenue Edouard Vaillant**
**F-18100 Vierzon (FR)**

(74) Mandataire: **Beauchamps, Georges et al**
**Cabinet Z.Weinstein 20, avenue de Friedland**
**F-75008 Paris (FR)**

Courier Press, Leamington Spa, England.

## Description

L'invention concerne une prothèse totale d'une articulation du corps humain telle que la hanche permettant l'intervention chirurgicale sur des patients présentant des usures, blocages, malformations, accidents ou maladies notamment de la hanche sans risque de luxation et à durée d'implantation prolongée.

Traditionnellement, les prothèses de l'articulation coxo-fémorale permettent la liaison rotule de la jambe et du bassin par l'intermédiaire d'une queue enfoncée et scellée dans le fémur, pourvue d'une calotte sphérique appelée tête fémorale et d'un cotyle scellé au moyen d'un ciment dans le bassin et comportant une calotte sphérique creuse qui reçoit la tête fémorale.

Le document FR-A-2 289 162 révèle une prothèse d'articulation de hanche comprenant un fût métallique 1 par exemple en alliage de titane scellé au moyen d'une colle 9 dans le fémur 8 et comportant, à son extrémité libre, un tenon conique 4 formant cône mâle 5 s'emmanchant dans le trou borgne conique 3 formant cône femelle d'une rotule 2 en céramique d'oxyde telle que l'alumine (notamment en poudre frittée) coopérant avec une cuvette artificielle par exemple en polyéthylène ou en métal destinée à être fixée dans le cotyle du bassin. Au lieu d'un assemblage à emmanchement conique avec effet d'autoblocage, la rotule peut aussi être collée sur le tenon cylindrique à l'extrémité libre du fût. Du côté opposé, le tenon s'élargit en forme de collet 7 s'appliquant sur la surface sectionnée du col du fémur.

Le document FR-A-2 310 120 concerne notamment la pièce fémorale d'une prothèse totale de la hanche, laquelle pièce comprend une tête sphérique 1 en alumine et une tige fémorale métallique 2 (en un alliage de titane à 6% d'aluminium et 4% de vanadium) se terminant par une extrémité mâle 6 raccordée par un col 3 au collet et s'engageant dans un trou borgne 4 de la tête sphérique. Cette extrémité mâle peut être fixée dans la tête soit par collage ou bien par fixation mécanique. Le trou borgne et l'extrémité mâle sont notamment tronconiques et l'extrémité mâle (à cône Morse) se fixe dans le trou par coincement. La tête sphérique comporte une face plane du côté de la tige.

Le document FR-A-2 437 200 révèle une endoprothèse 1 de la hanche, comprenant une hampe ou tige métallique 2 pour fixation fémorale endomédullaire et une tête sphérique 3 en céramique rigidement fixée à la tige. La partie saillante de la hampe se termine par une courte tige ou queue 4 présentant au moins une réduction de diamètre et s'emmanchant à force dans une cavité tronconique 12 de la tête sphérique. A l'embouchure de cette cavité, la tête sphérique comporte un plat 13. La courte tige extrême précitée se raccorde en s'évasant au reste de la hampe en se terminant par un collet 11 d'appui sur l'os fémoral sectionné.

Ces prothèses, bien qu'efficaces, présentent l'inconvénient de permettre des luxations de la hanche opérée surtout en période post-opératoire (6 mois) et ont une durée de vie réduite (10 ans) qui nécessite de nouvelles interventions plus délicates alors. Il s'ensuit que l'on cherche à retarder, au maximum, l'intervention, ce qui provoque un certain nombre de problèmes: — souffrances supportées par le patient — forte réduction de sa mobilité et de sa souplesse entraînant des arrêts de travail, des réductions d'activités, des dépenses de santé en médicaments anti-inflammatoires et analgésiques, en consultations médicales, rééducations, radiologies, cures, arrêts de longue maladie — des atteintes des autres éléments de la structure squelettique (colonne vertébrale, genou, cheville, pied) — des atteintes du système respiratoire et cardiovasculaire par manque d'activité physique et des problèmes secondaires de l'appareil digestif liés à la prise régulière de médicaments. Le patient, une fois opéré, ne souffre donc plus de sa hanche mais conserve toutes les atteintes à sa santé acquises dans la période pré-opératoire s'étalant sur plusieurs années (même jusqu'à 15 ans). Les deux documents antérieurs suivants préconisent respectivement des solutions techniques destinées à éviter ou à empêcher le risque de luxation précité.

Le document FR-A-1 047 640 révèle une prothèse d'articulation coxo-fémorale comprenant une rotule sphérique 1 comportant une queue 2 dégagée jusqu'à une embase 3 se prolongeant par une tige 4 destinée à être implantée jusqu'à l'embase dans le fémur dont le col a été scié. Cette rotule est destinée à pivoter dans une cuvette semi-sphérique 5 à fixer dans la cavité cotyloïde et de même diamètre que la rotule et pourvue de rainures de circulation de liquide organique sur sa face externe, la rotule étant maintenue dans sa cuvette par un dispositif de maintien destinée à éviter des luxations postopératoires et constitué par une bague ou collerette 8 vissée ou fixée de toute autre manière sur un collet 9 de la cuvette. La cuvette et la rotule sont en la même matière et la cuvette est revêtue intérieurement d'une garniture antifriction 10 par exemple en polyéthylène.

Le document US-A-2 947 308 révèle une prothèse complète de l'articulation de la hanche, comprenant une tête fémorale formant rotule sphérique 25, une cuvette hémisphérique 28 formant cotyle coiffant et s'articulant sur la rotule et fixée au pelvis 20, une corne arquée 26 fixée à la rotule 25 et destinée à pénétrer dans le fémur 49 dont le col a été scié, cette corne comportant un épaulement ou collet A venant en butée contre la face sciée du col et une bague annulaire formant couronne sphérique 5, 40 à bride 17, 42 fixée à la cuvette pour empêcher la luxation, cette fixation s'effectuant soit par rabattement de pattes 14, 15 de la cuvette autour de la bride de la bague ou par des rivets ou vis 47 soudés traversant des orifices correspondants de la bride de la bague et du rebord 12, 29 de la cuvette.

Ces dispositifs connus d'antiluxation présentent l'inconvénient d'être difficiles à fixer au cotyle artificiel et de nécessiter, pour cela, un mode opératoire d'exécution délicate. En outre, la fixation du cotyle artificiel dans l'acétabulum formant cavité cotyloïde de l'os coxal ou iliaque est relativement traumatisante pour ce dernier.

Pour permettre d'améliorer la longévité de la prothèse, les différentes fonctions du cotyle doivent être séparées. Le cotyle a, en effet, pour fonctions: — d'assurer la non-usure des surfaces frottantes et la réduction des efforts nécessaires au mouvement — d'encaisser les effets de chocs dus aux différentes activités de l'individu par effet d'amortissement — d'assurer la solidité et la longévité de l'ancrage du cotyle dans le bassin.

L'invention propose donc une prothèse de hanche du type comportant une corne destinée à pénétrer et à être scellée, au moyen d'un ciment, dans le fémur à col préalablement scié, et ayant un épaulement destiné à venir buter sur la face sciée du fémur, une tête fémorale sphérique liée à la corne, un cotyle destiné à être lié au bassin par un scellement et composé de deux coques métalliques sensiblement hémisphériques respectivement intérieure et extérieure entre lesquelles est interposé un matériau intercalaire élastique amortisseur de chocs.

Une telle prothèse de hanche est connue notamment par le document FR-A-2 266 491 ou par le document GB-A-2 126 096.

C'est ainsi que le document FR-A-2 266 491 concerne un ensemble orthopédique formant prothèse de l'articulation de la hanche, comprenant une broche métallique 1 scellée par un ciment ou une résine de scellement dans le fémur 19 et comportant un épaulement d'appui sur la face sectionnée du col du fémur ainsi qu'un pivot 2 s'emmanchant à frottement dans une cavité borgne 11-13 d'une rotule sphérique 5-7 destinée à pivoter dans un cotyle artificiel 14 scellé par un ciment ou une résine de scellement dans la cavité cotyloïde de l'os iliaque et composé d'une cage creuse hémisphérique métallique de roulement 15 garnie, sur sa face de contact glissant interne, d'un revêtement anti-usure 18 (par exemple en céramique), d'une cage métallique de scellement 16 à saillies d'ancrage sur sa périphérie externe et d'une couche intermédiaire de matériau amortisseur 17 notamment en silicone (hydrocarbure polymérisé) séparant les deux cages qui ainsi que la broche sont de préférence en titane.

Le document GB-A-2 126 096 concerne une prothèse d'articulation entre deux os et notamment de la hanche, à structure soit entièrement métallique ou partiellement métallique et partiellement céramique, comprenant une tige métallique à insérer et à sceller dans le fémur et à tête partiellement sphérique ou formant boule en métal ou en céramique, destinée à être reçue en contact pivotant glissant dans un cotyle artificiel 10 à surface concave interne ou cavité partiellement sphérique, à fixer par scellement dans l'acétabule formant cavité cotyloïde de l'os iliaque. Ce cotyle se compose de deux coques hémisphériques métalliques respectivement interne 31 et externe 35 séparées par une matière plastique élastique d'amortissement de chocs 32 interposée en sandwich sous pression et liée de façon adhérente à ces deux cuvettes ou coques métalliques minces 31, 35 qui comportent des rainures ou indentations 36 et/ou des saillies 37 sur leurs faces mutuellement en regard, de façon que les saillies pénètrent dans la couche intermédiaire de matière élastique et que les rainures soient remplies par celle-ci pour améliorer la liaison d'ancrage respectivement avec les deux cuvettes ou coques. La face extérieure de la cuvette ou coque externe 33 est pourvue de saillies 34 pour améliorer son scellement dans la cavité cotyloïde. La face de contact glissant interne de la coque interne 31 est revêtue d'une couche mince de céramique 35 telle que de l'alumine. La matière élastique intermédiaire peut être réalisée en un caoutchouc de silicone, en polyuréthane, en des fibres de carbone tissées et imprégnées ou en toute autre matière plastique élastique ou en un composé de caoutchouc.

Ces deux derniers documents antérieurs ne prévoient cependant aucun dispositif anti-luxation.

La présente invention a essentiellement pour but de remédier aux divers inconvénients affectant respectivement les divers dispositifs connus précités de la technique antérieure tout en combinant leurs différents avantages. Pour résoudre ce problème technique, l'invention a essentiellement pour objet une prothèse de hanche du type défini plus haut et qui est caractérisée par une contre-cuvette fendue, dans sa partie cylindrique parallèlement à son axe de révolution, à pattes élastiquement déformables pour être enclipsée sur la partie externe de la coque extérieure du cotyle et maintenue par un cerclage chirurgical disposé dans un logement formé dans la partie cylindrique de la contre-cuvette qui est ainsi centrée sur le cotyle, en ce que le matériau intercalaire est moulé sous pression et en ce que les coques comportent des rainures annulaires et méridiennes.

Le dispositif conforme à l'invention permet ainsi d'éviter les risques de luxation et de donner plus de latitude au chirurgien grâce à la contre-cuvette enclipsée sur la partie externe du cotyle artificiel et maintenue par un cerclage chirurgical par exemple en alliage de titane $TiAl_6V_4$ qui interdit, à la tête fémorale, de sortir de sa cavité et offre un appui complémentaire pour la réalisation de certains mouvements du patient ou pour la réduction de certaines malformations. Ce dispositif permet en outre d'améliorer la longévité de la prothèse grâce à la séparation précitée des différentes fonctions du cotyle. A cet effet, le cotyle se compose de plusieurs éléments et est constitué de deux coques recouvertes, sur leur face extérieure, une fois assemblées, de matériaux appropriés, ainsi que d'un matériau élastique injecté sous pression entre les deux coques. Les deux coques sensiblement hémisphériques sont en tôle métallique inaltérable, par exemple en alliage de titane $TiAl_6V_4$ et, par exemple, embouties et fluotournées pour être nervurées ou comporter des rainures annulaires et méridiennes réalisant les ancrages des autres matériaux du cotyle. Du fait du mode d'interposition (injection sous pression) du matériau intercalaire élastique formant ressort, les efforts, transmis par les coques métalliques, sont très bien répartis, ce qui autorise une élasticité importante du matériau médian qui amortit alors considérablement les effets de chocs, dus aux différentes activités de l'individu, qui ont tendance à détruire les liaisons osseuses nouvellement créées et la migration de l'os dans la matière des scellements et de la prothèse. La coque intérieure est recouverte, sur sa face concave, d'une couche ou pellicule de tout matériau approprié qui doit posséder une grande dureté, une grande résistance à la compression et au cisaillement, un bon ou très faible coefficient de frottement avec le matériau de la tête fémorale et permettre la lubrification du frottement. Des matériaux du type polyéthylène réticulé à haute densité, des matériaux frittés comme des carbures métalliques revêtus, des dérivés carbone-carbone ou des inclusions de graphite dans des carbures métalliques possèdent ces propriétés.

Les deux coques métalliques sont pourvues chacune, de préférence sur leurs deux faces opposées, de rainures annulaires et méridiennes pour assurer l'ancrage des éléments moulés ou frittés et des scellements. La surface externe ou convexe du cotyle peut être recouverte, dans une variante du procédé selon l'invention, par le même matériau ou un matériau hydroxyapatite et pourvu du même type de rainures que la coque externe afin d'assurer une meilleure liaison entre le scellement, l'os et la prothèse.

La corne métallique, par exemple en alliage de titane $TiAl_6V_4$, présente un épaulement qui appuie sur la surface sciée de l'os, après suppression de son col, une forme s'adaptant le mieux possible à la cavité interne ou endomédullaire du fémur (plusieurs formes et dimensions en fonction de la morphologie et de l'âge des patients) et une rugosité relativement importante de 0,1 à 0,4mm obtenue notamment par grenaillage, afin d'assurer la liaison dite "positive" entre le scellement et la prothèse sans pour autant risquer d'abîmer le fémur lors d'une dépose éventuelle.

La tête fémorale sphérique, en matériau ou à revêtement très dur, du type alumine $Al_2O_3$ par exemple, est soit frittée sur la corne qui comporte alors un bourrelet ou renflement d'ancrage de forme sphérique, soit mise en place sur la corne au moyen d'une liaison par adhérence. La forme extérieure de la tête fémorale est une portion de sphère d'angle au centre de préférence au moins égal à 270°.

Les différents matériaux utilisés doivent être compatibles entre eux et avec le corps humain (ni maladie provoquée, ni allergie, ni rejet).

L'invention sera mieux comprise et d'autres buts, caractéristiques, détails et avantages de celle-ci apparaîtront plus clairement à la lecture de la description explicative qui va suivre en se référant aux dessins annexés, donnés uniquement à titre d'exemples non limitatifs illustrant plusieurs modes de réalisation spécifiques actuellement préférés de l'invention et dans lesquels:

la figure 1 représente une vue en coupe longitudinale de l'appareil de prothèse totale de hanche implanté dans le fémur et dans la cavité cotyloïde de l'os iliaque du bassin, selon un premier mode de réalisation;

la figure 2 est une vue similaire isolée d'une variante d'exécution du dispositif complet selon la figure 1;

la figure 3 est une vue fragmentaire de détail, à plus grande échelle, de la tête fémorale selon la figure 2;

la figure 4 est une vue, semblable à la figure 2, d'une autre variante d'exécution du dispositif complet selon l'invention;

la figure 5 est une vue, semblable à la figure 3, d'un détail agrandi du dispositif selon la figure 4;

la figure 6 est sensiblement identique à la figure 5 mais sans le revêtement externe de la coque externe de la tête fémorale;

la figure 7 est une vue isolée en perspective d'une coque du cotyle artificiel représenté sur la figure 6;

la figure 8 est une vue éclatée en perspective d'un second mode de réalisation de l'appareil de prothèse totale selon l'invention, destiné à l'articulation scapulo-humérale de l'épaule;

la figure 9 est une vue isolée en perspective de la contre-cuvette; et

les figures 10 et 11 sont des vues semblables respectivement aux figures 4 et 5 mais illustrant encore une autre variante d'exécution.

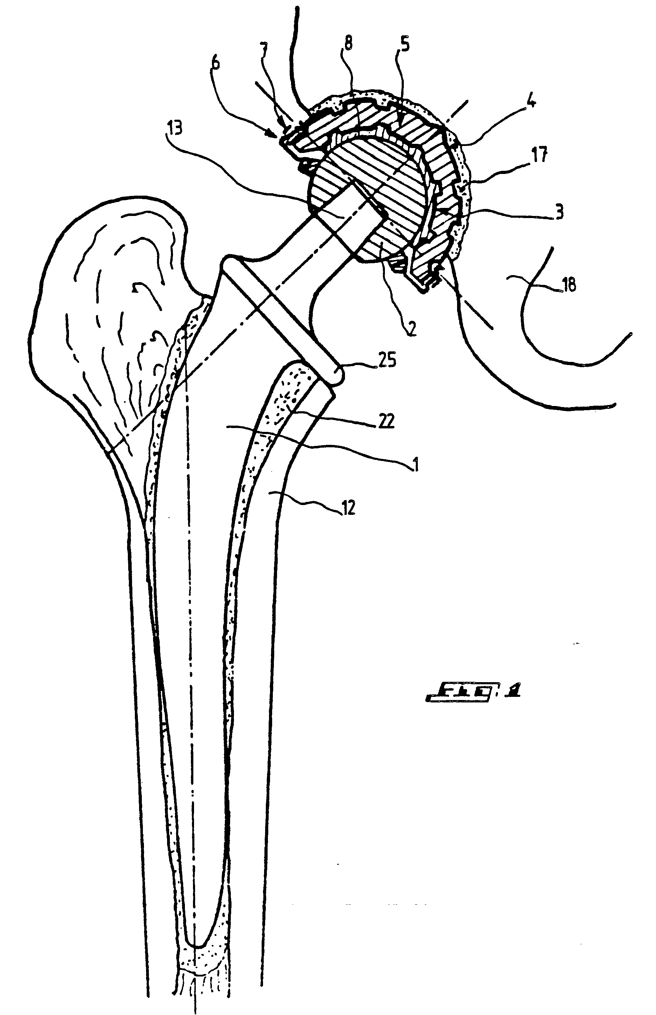

Le dispositif, représenté sur la figure 1, comporte une corne 1 à introduire dans le fémur 12 et de dimensions variables suivant les morphologies et l'âge des patients, sur laquelle est montée, par emmanchement en particulier, par coincement autoblocant sur un cône Morse 13, la tête fémorale 2. La corne comporte un épaulement ou collet précité 25. Le cotyle est constitué d'une coque intérieure 3 recouverte, sur sa face concave, de polyéthylène réticulé à haute densité par exemple 8 au contact de la tête fémorale 2, d'une coque extérieure 4 et d'un matériau élastique 5 moulé sous pression entre les deux coques. Une contre-cuvette 6, en tôle métallique (d'alliage $TiAl_6V_4$ par exemple) emboutie et fluotournée, est en forme de couronne annulaire fendue dans sa partie cylindrique parallèlement à son axe de révolution pour définir circonférentiellement des pattes ou languettes 14 (figure 9) par lesquelles elle vient s'enclipser sur la coque 4 et un cerclage chirurgical 7 empêche la contre-cuvette de se désenclipser. Les fentes ou pattes précitées assurent la déformabilité élastique de la contre-cuvette pour l'enclipsage.

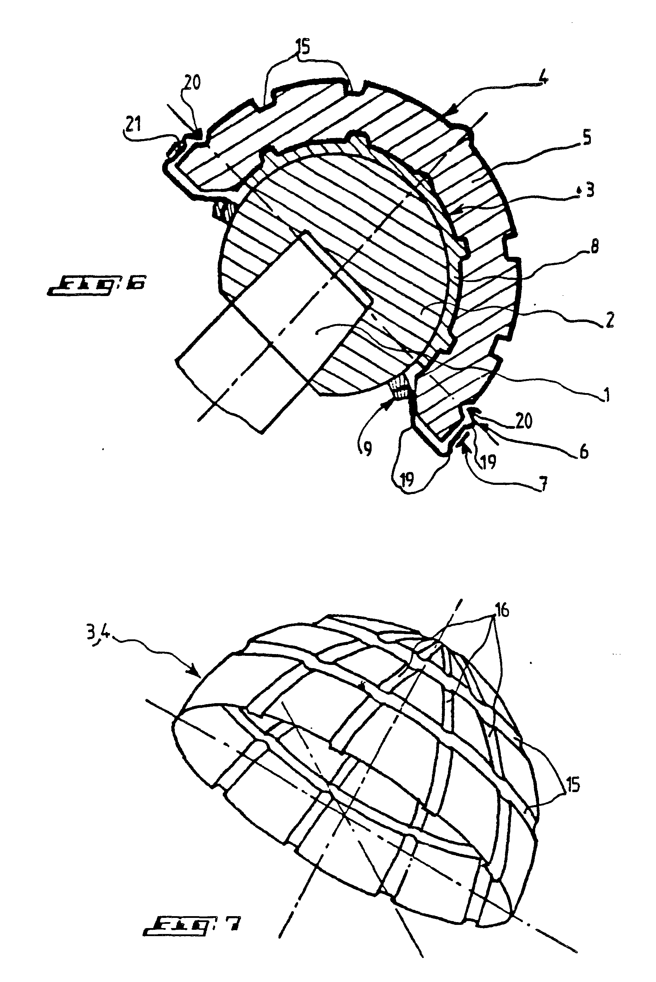

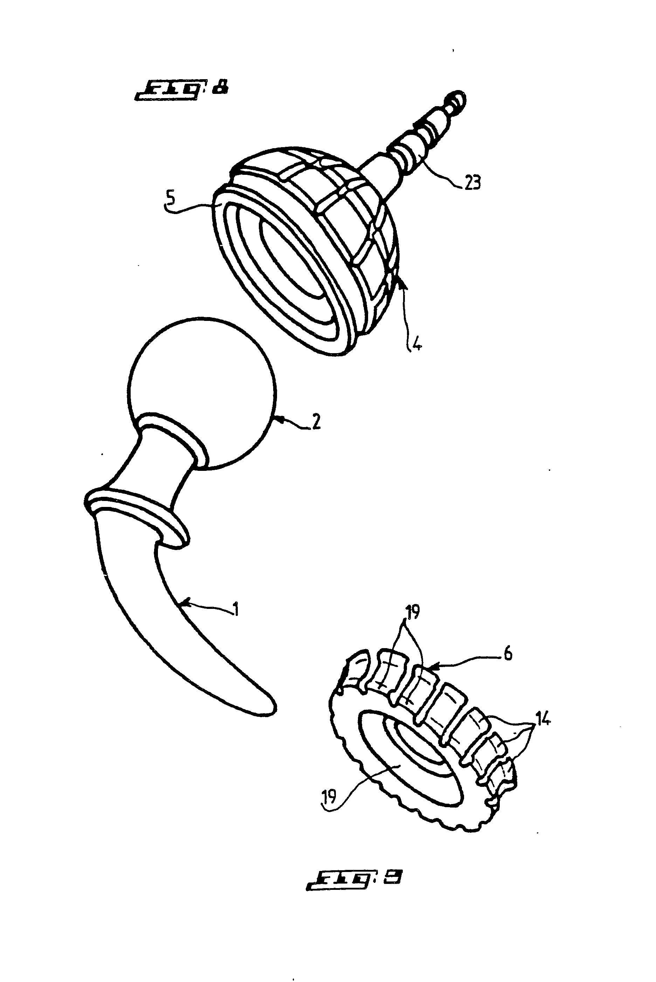

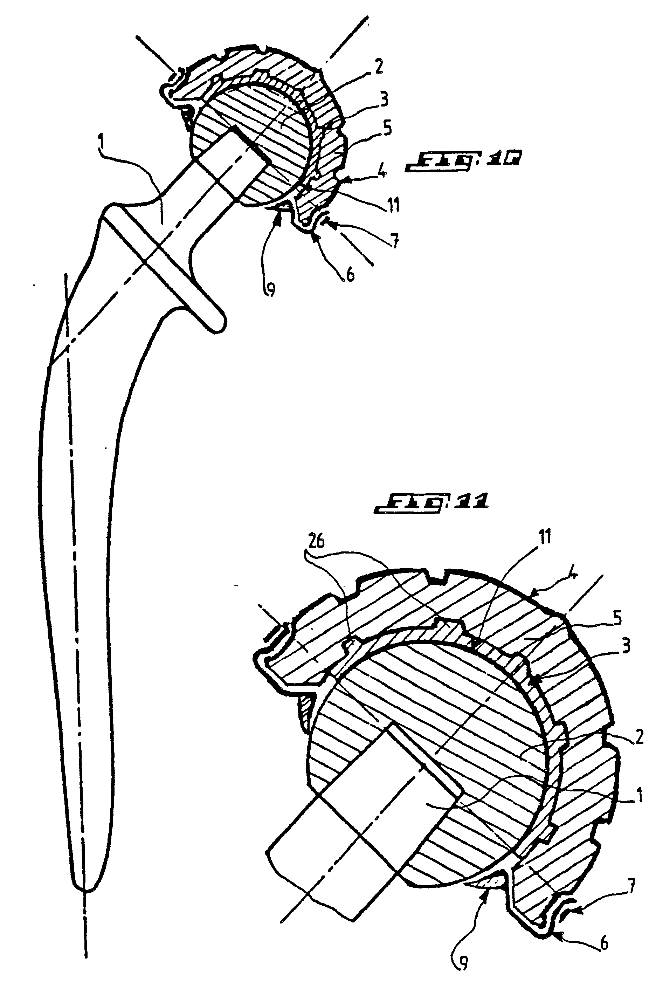

Les deux coques 3 et 4 sont munes de rainures respectivement parallèles et méridiennes 15, 16 (figures 6 et 7) permettant l'ancrage du matériau élastique 5, du polyéthylène réticulé à haute densité par exemple 8 et la liaison positive entre le scellement 17 du cotyle sur le bassin 18 et la coque externe 4.

La contre-cuvette 6 présente des plis 19 d'emboutissage assurant sa rigidité, ainsi que, dans sa partie cylindrique, une petite gorge 20 formant clips venant se loger dans une rainure annulaire 24 de la face extérieure de la coque externe 4 et une rainure formant logement 21 du cerclage chirurgical 7 afin d'éviter tout glissement de celui-ci parallèlement à l'axe de révolution de la contre-cuvette qui est ainsi centrée sur le cotyle et empêchée de se désenclipser de celui-ci; sa surface radiale vient s'appuyer sur le matériau élastique 5 pour lui conférer une bonne stabilité angulaire et assurer un meilleur positionnement de la surface de contact entre la contre-cuvette et la tête fémorale et une bonne stabilité de la contre-cuvette, cette surface et le bord radialement interne 9 de la contre-cuvette étant recouverts du même matériau que le revêtement intérieur de la coque interne 3. Les deux rainures 20 et 21 de la contre-cuvette 6 sont obtenues de préférence dans la même opération de fluotournage.

Le cotyle artificiel comporte la rainure annulaire 24 et un débordement des parties recouvertes extérieurement de forme cylindrique ou sphérique pour l'enclipsage de la contre-cuvette 6 sur la coque 4 du cotyle.

Lors de l'intervention chirurgicale, on met en place le cotyle, puis la corne fixée dans le fémur par un scellement 22; on introduit alors, autour de la partie saillante de la corne, la contre-cuvette et le cerclage chirurgical non monté; on met en place la tête fémorale, on emboîte la tête fémorale dans la cavité du cotyle, on enclipse la contre-cuvette et on met en place le cerclage chirurgical (tout ceci bien entendu suivant les règles de l'art et les pratiques chirurgicales bien connues).

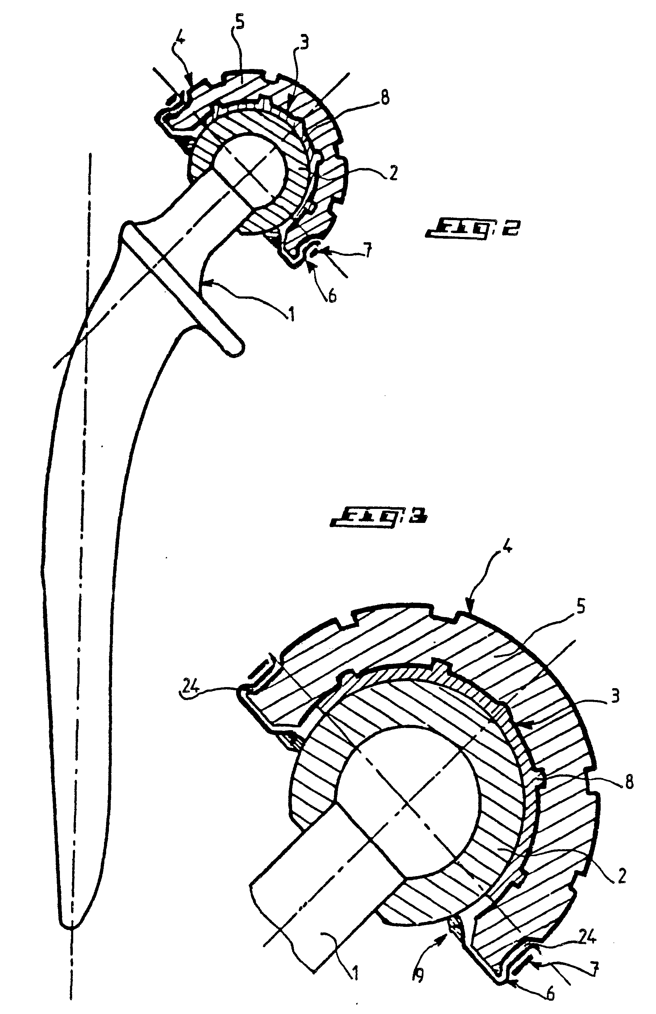

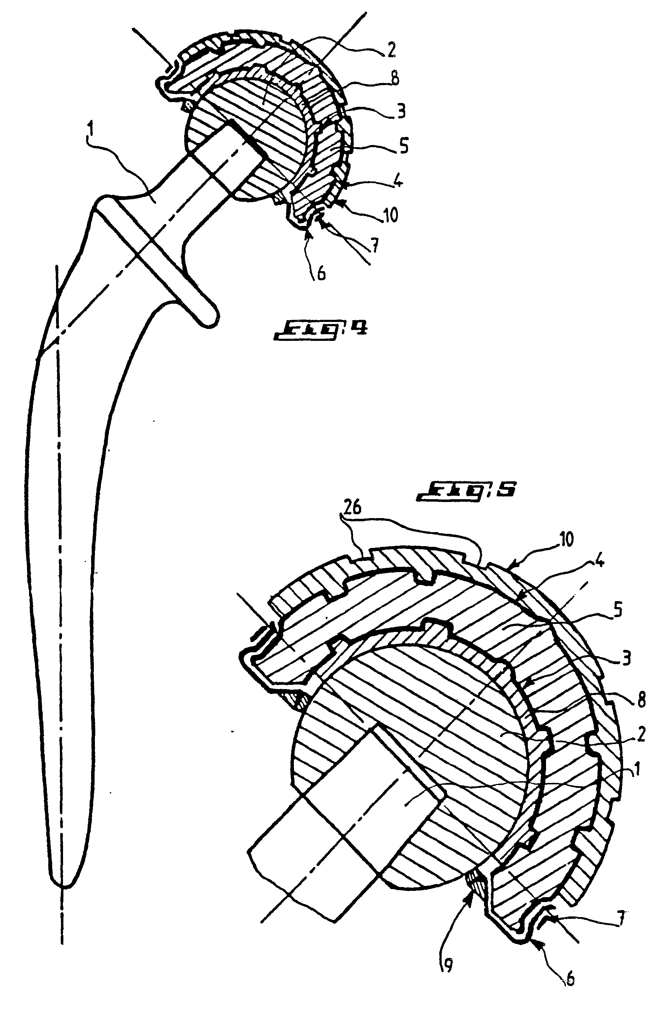

Le dispositif, représenté sur les figures 2 et 3, diffère du précédent par le fait que la tête fémorale est solidaire, de façon définitive, de la corne qui présente un bourrelet d'ancrage, que cett tête est obtenue par frittage de l'alumine sur la corne et que l'enclipsage présente une forme différente de celle de la figure 1.

Lors de l'intervention chirurgicale, il faut alors simplement introduire la contre-cuvette et le cerclage chirurgical par la pointe de la corne avant d'effectuer le scellement 22 de celle-ci dans le fémur, le reste de l'intervention restant inchangé par rapport au cas de la figure 1.

La prothèse, représentée sur les figures 4 et 5, réalise le revêtement 10, sur la face convexe de la coque extérieure 4, par du polyéthylène réticulé à haute densité ou un matériau hydroxyapatite par exemple, de préférence à rainures annulaires et méridiennes 26 assurant la liaison positive entre le cotyle et le scellement 17 de celui-ci dans le bassin 18. Ce dispositif permet d'allier la rigidité du contact au niveau du scellement, qui prolonge la vie de celui-ci, à une bonne adhésion du scellement, du matériau de contact de la prothèse et de l'os.

Cette prothèse peut être également utilisée dans le cas de prothèse d'épaule (figure 8) moyennant une légère modification de la forme extérieure du cotyle. La coque externe 4 présente alors une queue annelée 23 que l'on introduit et que l'on scelle par les procédés classiques connus dans la structure squelettique de l'épaule. La corne est alors scellée dans l'humérus et présente une forme appropriée à la morphologie squelettique de celui-ci.

Ce dispositif réduira le temps d'immobilisation dans le cas de scellements et de revêtement de la coque extérieure en dérivés de phosphates de calcium par exemple (matériaux hydroxyapatites) qui favorisent la colonisation osseuse de ceux-ci.

Le dispositif ainsi défini associe l'élasticité et la souplesse de la liaison comparable à celle de la hanche saine et la rigidité des surfaces de contact par le biais des coques métalliques, donc la longévité des scellements.

Cette élasticité et cette souplesse permettent, après l'intervention, de protéger les autres parties de la structure squelettique contre des chocs dus aux différentes activités du patient traité (même ceux dus à la simple marche), ce que ne permettent pas les autres tyoes de prothèses, en évitant ainsi la création d'arthroses secondaires de la cheville, du genou et de la colonne vertébrale.

Les figures 10 et 11 illustrent encore une autre variante de réalisation concernant en particulier les pièces réalisant le contact entre la partie fémorale et le cotyle artificiel du bassin ainsi que le dispositif anti-luxation.

Dans la prothèse précédemment décrite, la tête fémorale 2 est en alumine frittée et la surface de contact 8 est en alumine ou carbure métallique ou polyéthylène réticulé à haute densité, soutenue par une coque interne 3 en alliage de titene $TiAl_6V_4$ par exemple, nervurée, obtenue notamment per emboutissage et fluotournage et la contrecuvette 6, retenue par le cerclage chirurgical 7, comporte un recouvrement 9 de même metérieu que le revêtement 8 afin d'empêcher la luxation de la partie fémorale (la contre-cuvette 6 et le cerclage chirurgical 7 étant en le même matériau que la coque 3).

Cette solution peut éventuellement présenter les inconvénients suivants:

1) Si le matériau 8 est en polyéthylène réticulé à haute densité, il présente un bon coefficient de frottement avec l'alumine de la tête fémorale et une bonne adaptabilité des surfaces de 2 et 8 permettant de réduire les pressions de contact mais l'usure, due au frottement de glissement, peut être trop importante pour la longévité recherchée de la prothèse;

2) Si le matériau 8 est en carbure métallique ou en alumine, le problème de l'usure est résolu mais la rigidité des deux surfaces de contact glissant 2 et 8 fait que tout défaut géométrique, dimensionnel ou toute rugosité économiquement réalisable peut entraîner des pressions de contact trop élevées pour l'épaisseur du matériau 8 malgré la présence de la coque rigide intérieure 3;

3) En ce qui concerne le matériau 9, il peut empêcher tout mouvement de translation et créer des surcharges continuelles sur le cotyle artificiel et son scellement dans le bassin, ce qui peut limiter la durée de résistance du scellement.

Le perfectionnement selon les figures 10 et 11 a pour but de supprimer ces risques.

Il est préférable, et ceci réduit d'autant les coûts de production du cotyle artificiel, d'utiliser une coque interne 3 présentant des nervures radiales ou parallèles et méridiennes 26 sur sa face convexe, mais en étant lisse sur sa face concave et hémisphérique d'épaisseur plus importante (2 à 3 mm), forgée, traitée thermiquement et finement rectifiée sur sa face concave afin de pouvoir y déposer de l'or en déposant, par électrolyse, un film d'or 11 de quelque (1 à 3) centièmes de millimètre d'épaisseur et d'utiliser une tête fémorale 2 en un oxyde de chrome fritté ou recouvert par un oxyde de chrome par exemple par frittage et finement polie au lieu de l'alumine précédente, différents oxydes de chrome étant disponibles à cet effet.

Le couple or-oxyde de chrome possède, en effet, un très bon coefficient de frottement et la fine pellicule d'or permet l'adaptabilité des surfaces de contact en réduisant considérablement les pressions de contact effectives; la coque 3 plus épaisse assure la rigidité du film d'or et diffuse les efforts appliqués à celle-ci vers le matériau élastique 5 qui, du fait de la bonne diffusion ou répartition des pressions, de l'absence de mouvement relatif à sa surface, de la bonne cohésion des surfaces respectivement externe de la coque 3 et interne de la coque 4, obtenue par moulage sous pression du matériau élastique 5 et des nervures et rainures des deux coques ancrant ce matériau élastique, rend le fluage du cotyle tout à fait négligeable et ne transmet pas, dans le temps, de déséquilibre des efforts sur le scellement 17 du cotyle. L'épaisseur, plus réduite du matériau amortisseur 5 que dans des cotyles en polyéthylène classiques, reste supérieure à 6 mm et est largement suffisante puisque celui-ci ne reçoit que des pressions réparties et ne connait pas d'usure propre, du fait de l'absence de mouvement relatif à frottement d'éléments sur celui-ci.

En ce qui concerne le dispositif anti-luxation comprenant la contre-cuvette 6, le cerclage chirurgical 7 et le matériau 9, le perfectionnement réside dans le remplacement du matériau 9, tel que décrit précédemment ci-dessus, par une lèvre fine et souple, arquée ou de forme circulaire en polyéthylène qui, en permettant un léger déplacement ou une mobilité de la tête fémorale 2 dans le cotyle parallèlement à l'axe du col de la partie articulaire ou de la corne 1, limité, par la contre-cuvette, à une course de translation de 0,5 à 1 mm, permet, au dispositif anti-luxation, de n'agir qu'en cas de risque de luxation imminente et irréversible et donc principalement dans la période immédiatement post-opératoire. De ce fait, en utilisation normale, c'est la musculature seule du patient qui maintient la tête fémorale 2 dans son logement dans le cotyle sans transmettre, de façon continuelle, d'efforts d'arrachement au cotyle et à son scellement dans le bassin en restituant ainsi, à ce dispositif, toute son efficacité sans nuire à la longévité de la prothèse. La lèvre 9, venant s'appuyer légèrement sur l'arrière de la tête fémorale 2, sert à freiner la circulation du fluide corporel afin que celui-ci participe plus efficacement à la lubrification du contact à frottement entre la tête fémorale et la couche d'or 11 du cotyle.

Toutes les variations des solutions sont cumulables. Les différentes solutions réellement retenues peuvent présenter des variations par rapport aux solutions proposées sans pour autant sortir du cadre de l'invention tel que défini par les revendications. La prothèse selon l'invention permet l'utilisation de toutes les techniques opératoires, de tous les types de queues et de tous les scellement existants.

**Revendications**

1. Prothèse de hanche comportant: une corne (1) destinée à pénétrer et à être scellée, au moyen d'un ciment (22), dans le fémur (12) à col préalablement scié, et ayant un épaulement (25) destiné à venir buter sur la face sciée du fémur, une tête fémorale sphérique (2) liée à la corne (1), un cotyle destiné à être lié au bassin (18) par un scellement (17) et composé de deux coques métalliques sensiblement hémisphériques respectivement intérieure (3) et extérieure (4) entre lesquelles est interposé un matériau intercalaire élastique (5) amortisseur de chocs, caractérisée par une contre-cuvette (6) fendue, dans sa partie cylindrique parallèlement à son axe de révolution, à pattes (14) élastiquement déformables pour être enclipsée sur la partie externe (24) de la coque extérieure (4) du cotyle et maintenue par un cerclage chirurgical (7) disposé dans un logement (21) formé dans la partie cylindrique de la contre-cuvette (6) qui est ainsi centrée sur le cotyle, en ce que le matériau intercalaire est moulé sous pression et en ce que les coques (3, 4) comportent des rainures annulaires (15) et méridiennes (16).

2. Prothèse selon la revendication 1, caractérisée en ce que la contre-cuvette (6) comporte, dans sa partie cylindrique, une petite gorge (20) formant clips venant se loger dans une rainure annulaire (24) de la face extérieure de la coque externe (4).

3. Prothèse selon la revendication 1 ou 2, caractérisée en ce que la contre-cuvette (6) comporte des plis d'emboutissage (19) assurant sa rigidité et sa surface radiale vient s'appuyer sur le matériau élastique (5).

4. Prothèse selon l'une des revendications précédentes, caractérisée par la possibilité d'un léger déplacement ou d'une mobilité de la tête fémorale (2) dans le cotyle parallèlement à l'axe du col de la partie articulaire de la corne (1), limité par la contre-cuvette (6) à une course de translation de 0,5 à 1 mm.

5. Prothèse selon la revendication 4, caractérisée en ce que le bord radialement interne (9) de la contre-cuvette (6) est constitué par une lèvre fine et souple, arquée ou de forme circulaire en polyéthylène, venant s'appuyer légèrement sur l'arrière de la tête fémorale (2). (Figures 10 et 11).

6. Prothèse selon l'une des revendications précédentes, caractérisée en ce que les deux coques (3, 4) comportent des revêtements (8, 10) respectivement sur la face concave de la coque interne (3) et sur la face convexe externe de la coque externe (4), les rainures des coques servant d'ancrage aux autres matériaux ou éléments moulés ou frittés du cotyle et notamment du matériau élastique (5), des revêtements (8, 10) des coques (3, 4) et du scellement (17) entre cotyle et bassin (18).

7. Prothèse selon l'une des revendications précédentes, caractérisée en ce que tous ses matériaux sont biocompatibles et compatibles entre eux.

8. Prothèse selon l'une des revendications précédentes, à coques (3, 4) du cotyle et à corne (1) avec sa tête fémorale (2) en métal inaltérable, caractérisée en ce que la contre-cuvette (6) et le cerclage chirurgical (7) sont également en ce métal qui est l'alliage de titane TiAl$_6$V$_4$.

9. Prothèse selon l'une des revendications précédentes, caractérisée en ce que la coque intérieure (3) est revêtue, sur sa face concave, d'une couche ou pellicule de matière (8), dure, résistante à la compression et au cisaillement et à très faible coefficient de frottement au contact glissant avec la matière de la tête fémorale (2), telle que le polyéthylène réticulé à haute densité, des matériaux frittés tels que des carbures métalliques revêtus, des dérivés carbone-carbone ou des inclusions de graphite dans des carbures métalliques.

10. Prothèse selon l'une des revendications précédentes, caractérisée en ce que la surface convexe ou externe de la coque extérieure (4) est recouverte du même matériau (10) ou d'un matériau hydroxyapatite et ce revêtement est pourvu du même type de rainures (26) que la coque extérieure (4).

11. Prothèse selon l'une des revendications précédentes, caractérisée en ce que la surface et le bord radialement interne (9) de la contre-cuvette (6) sont recouverts du même matériau que le revêtement intérieur (8) de la coque interne (3).

12. Prothèse selon l'une des revendications précédentes, caractérisée en ce que la coque extérieure (4) à rainure annulaire (24) comporte un débordement des parties recouvertes extérieurement de forme cylindrique ou sphérique pour l'enclipsage de la contre-cuvette (6) sur cette coque externe (4).

13. Prothèse selon l'une des revendications précédentes, caractérisée en ce que la corne a un rugosité de 0,1 à 0,4 mm pour assurer une liaison positive avec le scellement (22) entre corne (1) et fémur (12).

14. Prothèse selon l'une des revendications précédentes, caractérisée en ce que la tête fémorale (2) a sa surface extérieure formée par une portion de sphère d'angle au centre d'au moins 270° et est en un matériau très dur tel que l'alumine Al$_2$O$_3$, qui est soit fritté sur un bourrelet ou renflement d'ancrage de la corne (1) ainsi rendue solidaire de la tête fémorale, soit la tête fémorale (2) est mise en place sur la corne (1) au moyen d'une liaison par adhérence en étant montée sur la corne (1) par emmanchement à coincement autobloquant sur un cône Morse (13).

15. Prothèse selon l'une des revendications précédentes, caractérisée en ce que cette coque intérieure (3) comporte de l'or (11) déposé sur sa face concave tandis que la tête fémorale (2) est en oxyde de chrome par exemple fritté et finement polie.

16. Prothèse selon la revendication 15, caractérisée en ce que la coque interne (3) a une épaisseur de 2 à 3 mm, comporte des nervures radiales ou parallèles et méridiennes (26) sur sa face convexe mais en étant lisse sur sa face concave qui est forgée, traitée thermiquement et finement rectifiée et revêtue d'un film d'or (11) de quelques centièmes de millimètre d'épaisseur, le matériau élastique (5) ayant une épaisseur supérieure à 6 mm.

17. Prothèse selon l'une des revendications précédentes, caractérisée en ce qu'elle est utilisable comme prothèse d'articulation scapulo-humérale d'épaule, la coque externe (4) comportant alors une queue annelée (23) destinée à être insérée et scellée dans la structure squelettique de l'épaule tandis que la corne (1) est scellée dans l'humérus, les scellements et le revêtement de la coque extérieure (4) étant de préférence en matériaux hydroxyapatites tels que des dérivés de phosphates de calcium (Figure 8).

**Patentansprüche**

1. Hüftprothese mit: einem hornartigen Schaft (1), der bestimmt ist, in den Oberschenkelknochen (12) mit vorher abgesägtem Hals einzudringen und mittels eines Zementes (22) eingekittet zu werden, und mit einer Schulter (25), die bestimmt ist, an die abgesägte Fläche des Oberschenkelknochens anzustossen, einen mit dem Schaft (1) verbundenen kugelförmigen Schenkelknochenkopf (2), einer Gelenkpfanne, die bestimmt ist, mit dem Becken (18) durch eine Verkittung (17) verbunden zu werden und aus zwei im wesentlichen halbkugelförmigen, metallischen jeweils inneren (3) und äusseren (4) Schalen besteht, zwischen welchen ein stossdämpfender, elastischer Zwischenwerkstoff eingesetzt ist, gekennzeichnet durch eine in ihrem zylindrischen Teil parallel zu ihrer Rotationsachse gespaltenen Gegenhülse (6), mit elastisch verformbaren Ansatzlappen (14), um über den Aussenteil (24) der Aussenschale (4) der Gelenkpfanne aufgeklemmt und durch eine in einer in dem zylindrischen Teil der Gegenhülse (6) gebildeten Ausnehmung (21) angeordneten chirurgischen Umschnürung (7) gehalten zu werden, wodurch die Gegenhülse an der Gelenkpfanne somit zentriert ist, dadurch dass der Zwischenwerkstoff unter Druck geformt wird und dadurch dass die Schalen (3, 4) Ringnuten (15) und Meridiannuten (16) aufweisen.

2. Prothese nach Anspruch 1, dadurch gekennzeichnet, dass die Gegenhülse (6) in ihrem zylindrischen Teil eine kleine klemmenförmige, in einer Ringnut (24) der Aussenfläche der Aussenschale (4) aufzunehmenden Rille (20) aufweist.

3. Prothese nach Anspruch 1 oder 2, dadurch gekennzeichnet, dass die Gegenhülse (6) ihre Steifheit gewährleistende Ziehfalten (19) aufweist und ihre Radialfläche zur Abstützung an dem elastischen Werkstoff (5) kommt.

4. Prothese nach einem der vorangehenden Ansprüche, gekennzeichnet durch die Möglichkeit einer leichten Verschiebung bzw. einer Beweglichkeit des Schenkelknochenkopfes (2) in der Gelenkpfanne parallel zur Halsachse des Gelenkabschnitts des Schaftes (1), welche durch die Gegenhülse (6) auf einen Translationsweg von 0,5 bis 1 mm beschränkt ist.

5. Prothese nach Anspruch 4, dadurch gekennzeichnet, dass der radial innere Rand (9) der Gegenhülse (6) durch eine dünne und nachgiebige, bogen- bzw. kreisförmige, sich leicht am Hinterteil des Schenkelknochenkopfes (2) abstüzenden Polyethylenlippe gebildet ist. (Figuren 10 und 11).

6. Prothese nach einem der vorangehenden Ansprüche, dadurch gekennzeichnet, dass beide Schalen (3, 4) Überzüge (8, 10) jeweils auf der konkaven Fläche der Innenschale (3) und auf der konwexen Aussenfläche der Aussenschale (4) aufweisen, wobei die Nuten der Schalen zur Verankerung der anderen geformten oder gesinterten Werkstoffen oder Elementen der Gelenkpfanne und insbesondere des elastischen Werkstoffes (5), der Überzüge (8, 10) der Schalen (3, 4) und der Verkittung (17) zwischen Gelenkpfanne und Becken (18) dienen.

7. Prothese nach einem der vorangehenden Ansprüche, dadurch gekennzeichnet, dass alle ihre Werkstoffe biokompatibel und miteinander kompatibel sind.

8. Prothese nach einem der vorangehenden Ansprüche, mit Schalen (3, 4) der Gelenkpfanne und mit Schaft (1) mit seinem Schenkelknochenkopf (2) aus unverderblichem Metall, dadurch gekennzeichent, dass die Gegenhülse (6) und die chirurgische Umschnürung (7) ebenfalls aus diesem Metall, welches die Titanlegierung TiAl$_6$V$_4$ ist, bestehen.

9. Prothese nach einem der vorangehenden Ansprüche, dadurch gekennzeichnet, dass die Innenschale (3) auf ihrer konkaven Seite mit einer Schicht bzw. Haut (8) aus einem harten, druck- und scherfesten Werkstoff mit sehr schwachem Reibungskoeffizienten bei Gleitberührung mit dem Werkstoff des Schenkelknochenkopfes (2), wie vernetzten Polyethylen hoher Dichte, gesinterten Werkstoffen wie überzogenen Metallkarbiden, Kohlen-kohlenderivate oder Graphiteinschlüssen in Metallkarbiden überzogen ist.

10. Prothese nach einem der vorangehenden Ansprüche, dadurch gekennzeichnet, dass die konvexe bzw. äussere Fläche der Aussenschale (4) mit dem selben Werkstoff (10) oder einem Hydroxyapatitwerkstoff überdeckt ist und dieser Überzug mit Nuten (26) der selben Gattung wie die Aussenschale (4) versehen ist.

11. Prothese nach einem der vorangehenden Ansprüche, dadurch gekennzeichnet, dass die Fläche und der radial innere Rand (9) der Gegenhülse (6) mit dem selben Werkstoff wie der Innenüberzug (8) der Innenschale (3) überdeckt sind.

12. Prothese nach einem der vorangehenden Ansprüche, dadurch gekennzeichnet, dass die Aussenschale (4) mit Ringnut (24) einen aus den aussen überzogenen zylinder- oder kugelförmigen Teilen hervorragenden Teil zum aufklemmen der Gegenhülse (6) auf diese Aussenschale (4) aufweist.

13. Prothese nach einem der vorangehenden Ansprüche, dadurch gekennzeichnet, dass der Schaft eine Rauheit von 0,1 bis 0,4 mm aufweist, um eine positive Verbindung mit der Verkittung (22) zwischen Schaft (1) und Oberschenkelknochen (12) zu gewährleisten.

14. Prothese nach einem der vorangehenden Ansprüche, dadurch gekennzeichnet, dass der Schenkelknochenkopf (2) seine Aussenfläche durch einen Kugelabschnitt mit Mittelpunktwinkel von wenigstens 270° gebildet hat und aus einem sehr harten Werkstoff wie Aluminiumoxid Al$_2$O$_3$ besteht, welcher entweder auf einem Verankerungswulst bzw. einer Verdickung des somit fest mit dem Schenkelknochenkopf verbundenen Schaftes (1) gesintert ist, oder der Schenkelknochenkopf (2) auf den Schaft (1) mittels einer Haftverbindung aufgesetzt wird, wobei er auf den Schaft (1) auf einen Morsekegel (13) durch Aufstecken mit selbsthemmender Verklemmung aufgesetzt wird.

15. Prothese nach einem der vorangehenden Ansprüche, dadurch gekennzeichnet, dass diese Innenschale (3) auf ihrer konkaven Seite abgelagertes Gold (11) aufweist, während der Schenkelknochenkopf (2) aus z. B. gesintertem und feinpoliertem Chromoxid gesteht.

16. Prothese nach Anspruch 15, dadurch gekennzeichnet, dass die Innenschale (3) eine Dicke von 2 bis 3 mm hat, Radial- bzw. Parallel- und Meridianrippen (26) auf ihrer konvexen Seite aufweist, jedoch glatt auf ihrer konkaven Seite ist, die geschmiedet, wärmebehandelt und feingeschliffen und mit einem Goldfilm (11) von einigen Hundertstel Millimeter Dicke überzogen ist, wobei der elastische Werkstoff (5) eine Dicke grösser als 6 mm aufweist.

17. Prothese nach einem der vorangehenden Ansprüche, dadurch gekennzeichnet, dass es als Prothese des Schultergelenkes zwischen Schlüsselbein und Oberarmbein verwendbar ist, wobei die Aussenschale (4) dann einen geringelten Zapfen (23) aufweist, der bestimmt ist, in das Schulterskelettgefüge eingesetzt und eingekittet zu werden, während der Schaft (1) in dem Oberarmbein eingekittet ist, wobei die Verkittungen und der Überzug der Aussenschale (4) vorzugsweise aus Hydroxyapatitwerkstoffen wie Derivate von Kalziumphosphaten bestehen (Figur 8).

## Claims

1. Hip prothesis comprising: a horn-shaped stem (1) adapted to penetrate and to be sealed by means of a cement (22) into the femur (12) the neck of which has been previously sawn off and having a shoulder (25) adapted to come into abutment upon the sawn face of the femur, a spherical femoral head (2) fast with the stem (1), a cotyle adapted to be connected to the pelvis (18) by a bedding (17) and consisting of two respectively inner (3) and outer (4) substantially hemispherical metallic shells between which is interposed an elastic, shock-absorbing intermediate material (5), characterized by a counter-cup (6) split in its cylindrical part in parallel relation to its axis of revolution, with elastically deformable lugs (14) for being clipped upon the external portion (24) of the outer shell (4) of the cotyle and maintained by a surgical wiring (7) arranged in a recess (21) formed in the cylindrical part of the counter-cup (6) which is thus centered upon the cotyle, in that the intermediate material is moulded under pressure and in that the shells (3, 4) comprise annular (15) and meridian (16) grooves.

2. Prothesis according to claim 1, characterized in that the counter-cup (6) comprises in its cylindrical part a small clip-like crease (20) to be accommodated in an annular groove (24) of the outer face of the outer shell (4).

3. Prothesis according to claim 1 or 2, characterized in that the counter-cup (6) comprises stamping folds (19) providing for its rigidity and its radial surface is caused to bear upon the elastic material (5).

4. Prothesis according to one of the foregoing claims, characterized by the possibility of a slight displacement or of a movability of the femoral head (2) in the cotyle in parallel relation to the axis of the neck of the articular portion of the stem (1), restrained by the counter-cup (6) to a translatory stroke of 0.5 to 1 mm.

5. Prothesis according to claim 4, characterized in that the radially inner edge (9) of the counter-cup (6) consists of a fine and flexible, arcuate or circularly shaped polyethylene lip caused to bear slightly upon the back of the femoral head (2). (Figures 10 and 11).

6. Prothesis according to one of the preceding claims, characterized in that both shells (3, 4) comprise linings (8, 10) on the concave face of the inner shell (3) and on the external convex face of the outer shell (4), respectively, the grooves of the shells serving as an anchoring for the other moulded or sintered materials or elements of the cotyle and in particular of the elastic material (5), the linings (8, 10) of the shells (3, 4) and of the sealing (17) between cotyle and pelvis (18).

7. Prothesis according to one of the foregoing claims, characterized in that all its materials are biocompatible and compatible between each other.

8. Prothesis according to one of the preceding claims, with shells (3, 4) of the cotyle and with the stem (1) having its femoral head (2) of a noncorrosive metal, characterized in that the counter-cup (6) and the surgical wiring (7) also are made from this metal which is the titanum alloy TiAl$_6$V$_4$.

9. Prothesis according to one of the foregoing claims, characterized in that the inner shell (3) is covered on its concave face with a layer or film of hard material withstanding compression and shear and having a very small coefficient of friction at the sliding contact with the material of the femoral head (2) such as high density reticulated polyethylene, sintered materials such as lined metallic carbides, carbon-carbon derivatives or graphite inclusions in metallic carbides.

10. Prothesis according to one of the preceding claims, characterized in that the convex or external surface of the outer shell (4) is covered with the same material (10) or with a hydroxyapatite material and this facing is provided with the same kind of grooves (26) as the outer shell (4).

11. Prothesis according to one of the foregoing claims, characterized in that the surface and the radially inner edge (9) of the counter-cup (6) are covered with the same material as the internal lining (8) of the inner shell (3).

12. Prothesis according to one of the foregoing claims, characterized in that the outer shell (4) with the annular groove (24) comprises a projection beyond the externally lined portions of cylindrical or spherical shape for the interlocking clipping of the counter-cup (6) onto this outer shell (4).

13. Prothesis according to one of the preceding claims, characterized in that the stem has a rugosity of 0.1 to 0.4 mm to provide for a positive connection with the sealing (22) between stem (1) and femur (12).

14. Prothesis according to one of the preceding claims, characterized in that the femoral head (2) has its outer surface formed of a portion of a sphere containing an angle at the centre of at least 270° and is of a very hard material such as alumina Al$_2$O$_3$ which is either sintered onto a boss or bulge for anchoring the stem (1) thus made fast with the femoral head, or the femoral head (2) is positioned on the stem (1) by means of a bonding through adherence by being mounted on the stem (1) by being fitted with a self-locking wedging onto a Morse cone (13).

15. Prothesis according to one of the foregoing claims, characterized in that this inner shell (3) comprises gold (11) deposited on its concave face whereas the femoral head (2) is made from for instance sintered and finely polished chromium oxide.

16. Prothesis according to claim 15, characterized in that the inner shell (3) has a thickness of 2 to 3 mm, comprises radial or parallel and meridian ribs (26) on its convex face but while being smooth on its concave face which is forged, thermally treated and finely ground and covered with a gold film (11) with a thickness of a few hundreds of a millimeter, the elastic material (5) having a thickness above 6 mm.

17. Prothesis according to one of the foregoing claims, characterized in that it is usable as a prothesis of the scapulo-humeral shoulder joint, the outer shell (4) then comprising a ringed shank (23) adapted to be inserted and bedded into the squelettal structure of the shoulder whereas the stem (1) is bedded in the humerus, the beddings and the lining of the outer shell (4) being preferably made from hydroxyapatites materials such as derivatives of calcium phosphates (Figure 8).

FIG 1

EP 0 208 578 B1

_Fig 2_

_Fig 3_

2

EP 0 208 578 B1

_FIG_ 4

_FIG_ 5

3

15

20

21

4

5

3

8

2

1

Fig. 6

20

6

9

19

19

7

3,4

16

15

Fig. 7

4

FIG. 8

FIG. 9

FIG. 10

FIG. 11